# EUROPEAN PATENT APPLICATION

(11) **EP 1 363 224 A1**
(43) Date of publication of application: **19.11.2003**
(21) Application number: 02010817.1
(22) Date of filing: 15.05.2002
(51) Int. Cl.: G06F 19/00, A61J 7/00

(54) **A system for monitoring medical data, a terminal device for measuring and storing medical data, a medicine container and a holder for medicine containers**

(71) Applicant: Microlife Intellectual Property GmbH, 9442 Berneck (CH)
(72) Inventor: Lin, Kin-Yuan, Nei-Hu, Taipei 114 (TW); Brunvoll, Morten, 9442 Berneck (CH)
(74) Representative: Hepp, Dieter

(57) **Abstract**

A system for monitoring medical data of a patient comprises at least one central computer (10) and at least one terminal device (20) for measuring and storing medical data (D). The terminal device (20) has a communication interface (23) for exchange of data (S, D, C) with the central computer (10). Computer readable program means (S) can be loaded from the central computer (10) into the terminal device (20).

## Description

The invention relates to a system for monitoring medical data, a terminal device for measuring and storing medical data, a medicine container and to a holder for medicine containers.

Systems for monitoring medical data are known in a wide variety. The need for monitoring medical data such as blood pressure, glucose level, or the weight has been widely recognised. It is also recognised that continuous monitoring of such parameters is important in view of the control of a medical treatment. There are a number of proposals for systems allowing monitoring of medical data and monitoring of a patient's compliance with a prescription of medicine.

In the European patent application EP-301 672A2 an interactive drug dispenser has been proposed. The drug dispenser actively controls the manner in which medicine is administered to a patient.

EP-370 599B1 discloses a medicine container which is provided with a drug dispensing event detector. Removal of medicine from the container is recorded by detection of cap openings of the container.

US-4 933 873 discloses an interactive patient assistant device, which includes both preselected doses of medication and a physical testing device. The device keeps track of medication and diagnostic testing schedules. The device makes a preselected dose of medication available to the patient. The system can communicate with a remote medical center over the telephone system by way of a modem.

US-5 016 172 discloses a system for monitoring patient compliance and patient status. Medicine consumption as well as physical parameter such as heart rate or the like are recorded. Those parameters are transmitted from time to time to a central computer and optionally to a remote computer for professional or hospital use.

US-5 710 551 discloses a system for remote monitoring of self-medication of a patient to assure compliance with a prescribed dosage schedule. The system may be connected to a remote central monitoring station via a communications link.

WO98/49659 discloses a prescription compliance device and method. A patient is reminded when the next dose of medication is to be taken and indicates whether a specified dose has been taken.

WO00/32088 discloses a medical system and a method of controlling the system for medical self-treatment. The system consists of several portable modules. One module is designated as a master module, which controls, supervises and monitors information and data exchange between itself and the rest of the modules. The modules may consist of a measuring device (blood glucose monitor) or of a doser, an inhaler, a tablet dispenser and storage container.

WO00/32097 discloses a system and a method for executing a treatment regimen. Medical personnel can receive feedback from the patient regarding the medical regimen. A medicine dispenser is coupled to a client device. The medicine dispenser dispenses only the dosage of medicine directed by this prescribed medical regimen and records information from the patient regarding compliance.

US-5 967 975 discloses a system for the automated remote monitoring of home health parameter measurement activities to assure outpatient subscriber compliance with a prescribed measurement schedule regimen. The monitored health parameters typically included blood pressure, blood glucose level, clotting factor and the like.

EP-1 034 734 discloses a device for displaying a series of medication compliance and health parameter data. The device allows to make a correlation between medicine compliance and health parameters. A report generated by the device may be printed out in a hard copy.

US-4 837 719 discloses a device for producing a report each time when medicine is taken by a patient. Such report is printed by means of a printer. In addition, the device may be used to monitor vital signs of the patient, such as blood pressure.

Despite the large number of systems proposed for monitoring patient data and patient compliance, such devices or systems are not used on a wide scale in current health care systems. There are a number of drawbacks to those known systems. A first drawback is, that many of such systems require personal computers at the patients locations. Patients are, however, often not used to work with computers. An additional drawback is, that such systems, which often include medical parameter monitoring devices such as blood pressure monitors are relatively expensive. Often, such systems are considered to be too expensive by users wanting to make a first routine check. In addition known medical parameter measuring devices such as conventional blood pressure monitors have standard software programs installed therein. In many cases, such standard software does not perfectly fit the needs of a specific patient.

A further drawback of systems allowing the monitoring of patient compliance is, that pillboxes proposed in such systems are unhandy or that handier pillboxes do not allow to store medicine for a longer period of time and therefore do not allow monitoring of medicine complience over such longer periods of time.

It is therefore an object of the present invention to avoid the drawbacks of the prior art. A first object of the invention especially consists in providing a system for monitoring medical data which does not necessitate a personal computer at the patients premises. A second object of the invention consists in providing a system and a device for monitoring medical data of a patient which can be used by a plurality of users. The same device should be subsequently usable by several users who have not yet decided to personally acquire such a device. Still a further object of the invention consists in providing a device for monitoring medical data which can be specifically adapted to the patients needs. A further object of the invention consists in providing a medicine container which is handy, and which allows the storage of medicine which should be taken over a relatively long period of time. The medicine container should further allow a reliable monitoring of the patient compliance.

These and other objects are solved with a system for monitoring medical data of a patient, a device for monitoring and storing medical data, a medicine container and a holder for a medicine container according to the independent patent claims.

The system comprises at least one central computer. The central computer has a first communication interface. The system further comprises at least one terminal device for measuring and storing medical data of a patient. The terminal device is originally formed without any specific software for measuring and storing medical data. The terminal device has a calculating arrangement with means for storing computer readable program means. The computer readable program means cause the calculating arrangement to acquire, treat or store medical data. The terminal device is further provided with a second communication interface allowing the exchange of data. Computer readable program means are stored on the central computer. The program means can be loaded from the central computer into the means for storing of the terminal device by means of the first and second communication interfaces. The invention provides a terminal device which is basically free from specific program code for acquiring, treating or storing data. Appropriate program means can be loaded into the terminal device from the central computer according to a patient's need.

The terminal device is provided with software code of an operating system for operating the terminal, but without any software instructions relating to the acquisition, storage and treatment of medical data. Individual program code means are loaded into the terminal device for each individual user. As compared to conventional medical parameter monitoring devices such as conventional blood pressure monitors, the terminal device according to the present invention allows to load a computer program for data acquisition, treatment and storage and also allows to delete such computer program by means of a communication interface which is directly accessible e.g. by a conventional computer provided with an infrared communication interface.

The central computer can be typically a personal computer located at a pharmacy or at a doctor's office. Such a system has a number of advantages. A first advantage is, that user specific programs can be loaded into the terminal device. Depending on the age, risk factors or other patient specific parameters, different data acquisition or treatment modes can be selected. There is no need for the user to select among or plurality of such programs as the appropriate program is directly stored into the terminal device. A further advantage is, that it is possible to always use the most recent version of the computer program for operating the terminal device. Such a terminal device may be used by a plurality of users. The provider of a system, e.g. a pharmacist can rent such a terminal device to different users for test purposes. At the beginning of a test phase, an appropriate computer program is loaded into terminal device.
The central computer is typically formed as a conventional personal computer. The system further may include one central server. The central server may be especially used for centrally storing the computer readable program means. The central computer is adapted to access the central server for downloading the centrally stored computer readable program means. The provider of the medical data monitoring system can store the most recent versions of the computer program of the central server. If the software is downloaded from the central server to the central computer and then to the terminal device, it is made sure that only the most recent version of the software will be used.

The central computer can be further adapted to receive medical data acquired or stored in the terminal device. Transmission of data can be also made by means of the communication interfaces provided in the central computer and in the terminal device.

According to a further preferred embodiment of the invention, the computer readable program means are designed to run on the terminal device only for a predetermined period of time. Such a system is especially designed for evaluation purposes. A patient intending to continuously monitor a health parameter such as his or her blood pressure can rent such a terminal device for a period of time. The provider of the system loads a software corresponding to the patients needs into the terminal device. The software is operatable during the desired evaluation period. At the end of the evaluation period, the patient returns the terminal device to the provider of the medical data monitoring system.

In a further preferred embodiment of the invention, the central computer is adapted to treat the medical data received from the terminal device. The central computer may generate a report relating to the medical data. When the patient returns the terminal device to the provider of the system, medical data stored in the terminal device are transmitted to the central computer and are stored and treated therein. A patient specific report may be printed out in the central computer. Such report may be the basis for advising the patient. The report could e.g. advise the patient to see a doctor, to change his lifestyle or the like.

According to a further preferred embodiment of the invention, the terminal device has at least one connector for removably connecting an external sensor for a measuring the medical data. If the terminal device is used for a plurality of different users, there may be hygienic concerns. The terminal device is particularly adapted as a blood pressure monitor. Conventional blood pressure monitors comprise a cuff which is adapted to be wrapped around a patients upper arm or wrist. Such cuffs need to be carefully cleaned when the terminal device is returned by the patient. According to this preferred embodiment of the invention, the cuff can be removed from the terminal device and can be replaced by a new or a cleaned cuff. According to a preferred embodiment of the invention, disposable cuffs can be used for that purpose.

The terminal devices can further be adapted to receive data from additional medical data monitoring devices. It is e.g. possible to send patient compliance data from a medicine container to the terminal device and to store such compliance data in the terminal device. Other medical data monitoring devices such as a body weight scale or a glucose meter could also be used for sending data to the terminal device. When the terminal device is returned to the provider of the system, all data stored therein can be transmitted to the central computer.

According to still a further embodiment of the invention, the terminal device is provided with a touch screen. This touch screen allows the entry of user instructions and the display of medical data. As a plurality of different software instructions could be loaded in the terminal device, a plurality of different user input interfaces may be necessary. A touch screen allows to provide different entry fields for user instructions on a software basis. Such a device thus can be provided with only one physical operation button. The single operation button is only used to switch on/off the device. All other user instructions can be entered through the touch screen.

The system according to the invention can further comprise at least on portable medicine container. The portable medicine container comprises a third communication interface. The third communication interface is adapted to exchange medicine compliance data with the communication interface of the terminal device.

Medicine compliance data are thereby stored in the terminal device. Medicine compliance data can be transferred to the central computer when the medical parameter data are transferred to the central computer. Such a system provided with a medicine container allows to monitor the effectiveness of a medical treatment as a correllation between medical parameters and the medicine which has been taken by the patient can be made.

According to a further preferred embodiment of the invention, the medicine container comprises a housing and a drawer for the reception of medicine. The drawer is insertable into the housing. The medicine container is provided with means for detecting the withdrawal of the drawer from the housing. According to still a further embodiment of the invention, the medicine container is provided with an alarm means for reminding the patient to take his or her medicine. Alternatively, the drawer can be provided with means e.g. optical or mechanical sensors for detecting removal of medicine from the drawer.

According to a further preferred embodiment of the invention, the system is additionally provided with a holder for receiving a plurality of drawers for such a medicine container. In each drawer, medicine for e.g. one day can be stored. The holder allows to store medicine for e.g. one week. The drawers held in the holder can be filled with medicine in a pharmacy. The patient removes a drawer from the holder in the morning of every day. The patient then inserts the drawer into the medicine container. Each time, the drawer is removed from the medicine container and medicine is taken, corresponding compliance data are stored in the medicine container. At the end of the day, the drawer is removed from the housing of the medicine container and placed back in the holder and the next drawer is inserted into the medicine container. Compliance data stored in the medicine container may be transmitted to the terminal device when the drawer is removed from the medicine container. Alternatively it is also conceivable to detect compliance of medicine in the holder when the drawer is put back in the holder. Medicine compliance data subsequently can be transmitted from the holder to the terminal device.

According to the invention, there is also provided a terminal device as described above. Each system may have a plurality of terminal devices. The invention provides a medicine container as described above and a holder for a plurality of drawers of such medicine containers. The invention further provides a method for providing a medical parameter measuring device by loading appropriate Software in a terminal device.

The invention will be described in more detail by way of embodiments shown in the following drawings. There are shown:
- Figure 1:: A schematic representation of a system according to the invention,
- Figure 2a,b,c:: a schematic representation of data transfer in a terminal device according to the invention,
- Figure 3a,b:: a three dimensional representation of a terminal device with and without a medical parameter measuring sensor,
- Figure 4a,b:: a three dimensional representation of a medicine container with an inserted medicine drawer and with a removed medicine drawer, and
- Figure 5a,b:: a three dimensional representation of a holder for drawers of a medicine container.

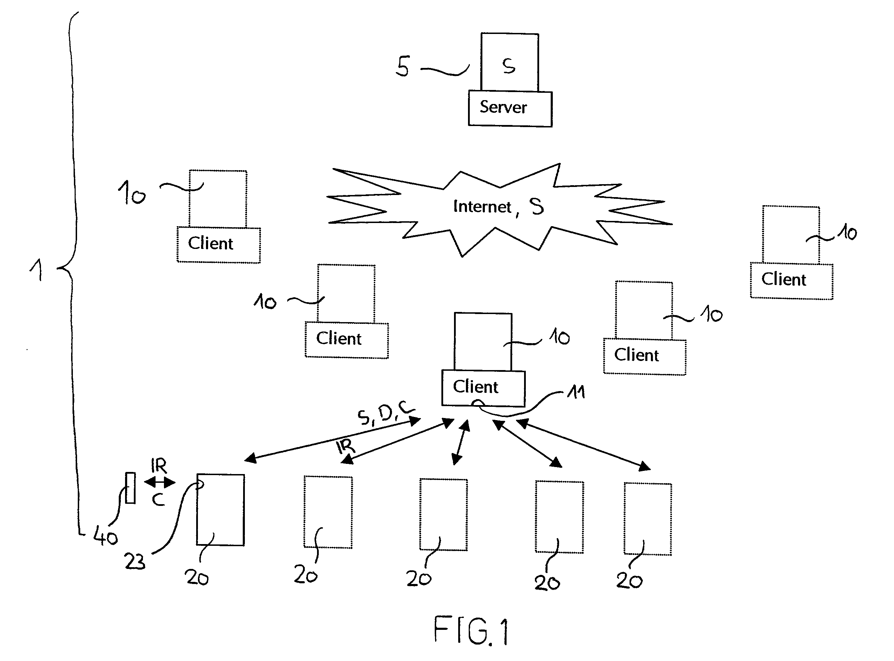

Figure 1 schematically shows a system 1 according to the present invention. The system 1 comprises a plurality of terminal devices 20. The terminal devices 20 are provided with a sensor for measuring a medical parameter (not shown) and with an infrared communication interface 23. The communication interface 23 is adapted to exchange data with a central computer 10. The central computer 10 is also provided with an infrared communication interface. The terminal device further communicates with a medicine container 40 over an infrared communication IR. The system 1 comprises a plurality of further central computers 10. Each of the furhter central computers 10 (shown in dotted lines) can also communicate with a plurality of terminal devices 20.

The central computers 10 are formed as web-clients which communicate with a central server over a computer network such as the internet.

The central server 5 is operated by the provider of the system 1. Software programm means S are stored in the central server. The software S can be downloaded to the central computer 10 over the internet. The software can be loaded from the central computer 10 to the terminal device 20 over the infrared communication IR. If the software is loaded into the terminal device 20, the terminal device operates as a conventional medical parameter monitoring device, e.g. as a blood pressure monitor. The software causes the terminal device 20 to acquire medical parameters, to store the medical parameters in the terminal device 20 and also to transmit the medical data D to the central computer 10 if required.

Compliance data C are transmitted from the medicine container to the terminal device 20 and temporarily stored therein. The compliance data are then transmitted from the terminal device 20 to the central computer 10.

The system 1 is especially suitable for a health management system. A provider which operates the server 5 is also providing central computers 10, terminal devices 20 and medicine containers 40. The system 1 is locally provided e.g. by a pharmacist which uses one of the central computers 10. Terminal devices 20 are rented to patients for a certain period of time. At the beginning of this period of time, an appropriate software S is loaded into the terminal device 20. At the end of this trial period, this software is desactivated in the terminal device 20 and the patient returns the terminal device 20 to the pharmacist. Medical data D and also patient compliance data C are loaded to the central computer 10.

Patient specific softwares S can be loaded into the terminal device 20. It is e.g. possible to load specific patient compliance software as disclosed in the co-pending PCT-Application PCT/EP01/15306 or software allowing a stratification of individual risks of a patient as disclosed in co-pending application EP 01121541.

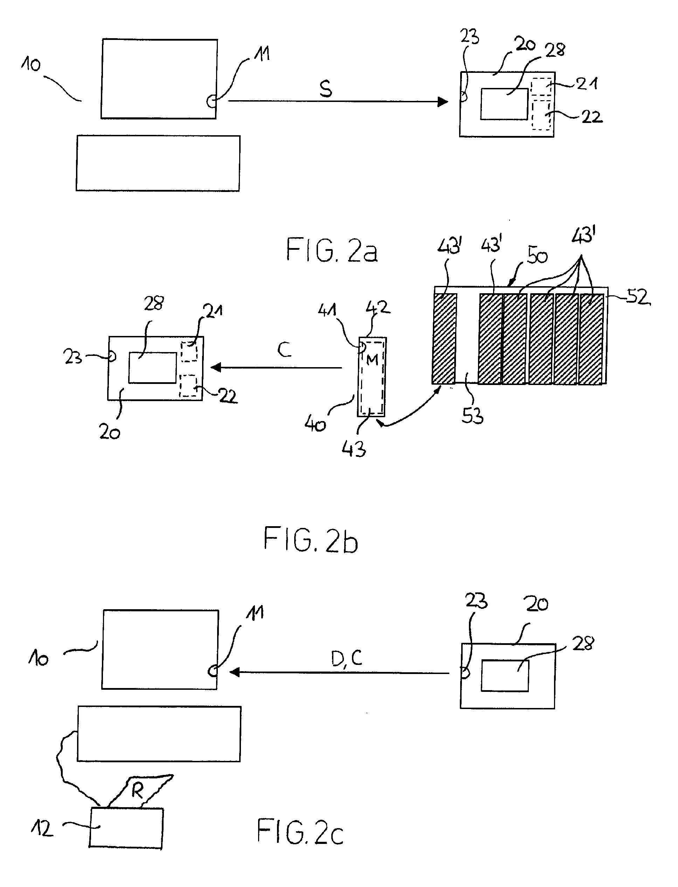

The different ways of communication of data of the terminal device are shown in figures 2a to 2c. Figure 2a schematically shows downloading specific software code S from a central computer 10 into the terminal device 20. For downloading the software S, an infrared communication interface 11 on the central computer and an infrared communication interface 23 on the terminal device are used. Of course, other communication means such as wire communication with serial ports or a USB port of a computer or other wireless communication such as communication with the blue-tooth technology are conceivable. The terminal device 20 comprises a calculating arrangement 21 and storage means 22. The calculating arrangement 21 and the storage means 22 are formed as conventional storage means such as flash cards and of a conventional micro processor. Other storage means are conceivable. The terminal device 20 is further provided with a touch screen 28 allowing the display of medical parameter data and also allowing the user to input specific user instructions. Once the software S is loaded into the terminal device 20, the terminal device 20 can be operated as a conventional medical parameter measuring device.

Figure 2b shows transfer of patient compliance data C from a medicine container 40 to the terminal device 20. The medicine container 40 is provided with an infrared communication interface 41. The medicine container consists of a housing 42 in which a drawer 43 is inserted. Medicine, e.g. pills is stored in the drawer. The system further includes a holder 50 for holding a plurality of drawers 43. The holder is provided with a housing 52, in which channels 53 for the drawers are formed. The holder 50 can be e.g. provided with seven channels 53. One drawer per day may be stored in the holder 50. Patient compliance data C e.g. can be transmitted from the medicine container 40 to the terminal device 20 each time when the drawer 43 is completely removed from the housing 42 and is replaced with a new drawer 43' filled with medicine.

Figure 2c shows data communication between the terminal device 20 and the central computer 10. Medical parameter data D stored in the terminal device 20 and patient compliance data C stored in the terminal device 20 are transmitted to the central computer 10. The central computer 10 is provided with a printer 12 for printing out a report R relating to the medical parameter data D and patient compliance data C. Transfer of data D, C from the terminal device 20 to the central computer 10 is made at the end of a trial period, when the software S stored in the terminal device 20 has been automatically desactivated and when the terminal device is returned e.g. to the pharmacist.

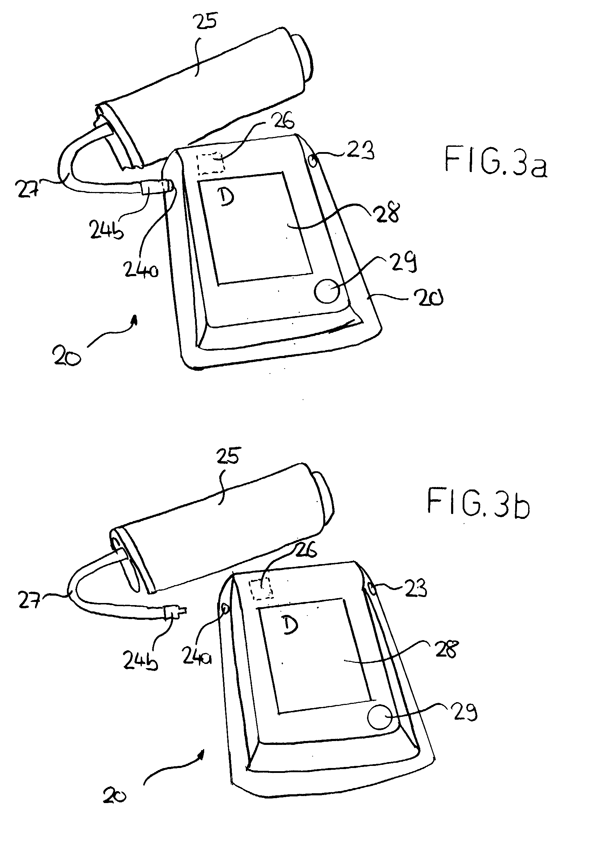

Figures 3a and 3b show a typical embodiment of a terminal device 20 formed as a blood pressure monitor. The terminal device 20 is provided with a connector 24a for receiving a connector 24b attached to a tube 27 of a cuff 25 for measuring the blood pressure. The cuff 25 is adapted to be wrapped around a patient's wrist or upper arm in a conventional manner. The terminal device 20 is provided with a pressure sensor 26 for measuring the pressure in the cuff. The connectors 24b, 24a allow to removably connect the tube 27 to the terminal device 20. The terminal device 20 therefore can measure the pressure in the cuff 25 by means of the pressure sensor 26. The terminal device 20 is further provided with a on/off button 29. The button 29 is used to turn the terminal device 20 on or off. All other user instructions are entered into the terminal device by the touch screen 28. Medical parameter data D such as the heart rate, the diastolic or the systolic blood pressure are also displayed on the touch screen 28. Medical data D additionally are stored in storage means 22 of the terminal device 22. If during the trial period the patient is asked to measure his or her blood pressure three times a day, data relating to three measurements are stored in the storage means 22 every day. The data are transmitted to the central computer 10 as shown in figure 2c after the end of the trial period which may be e.g. thirty days.

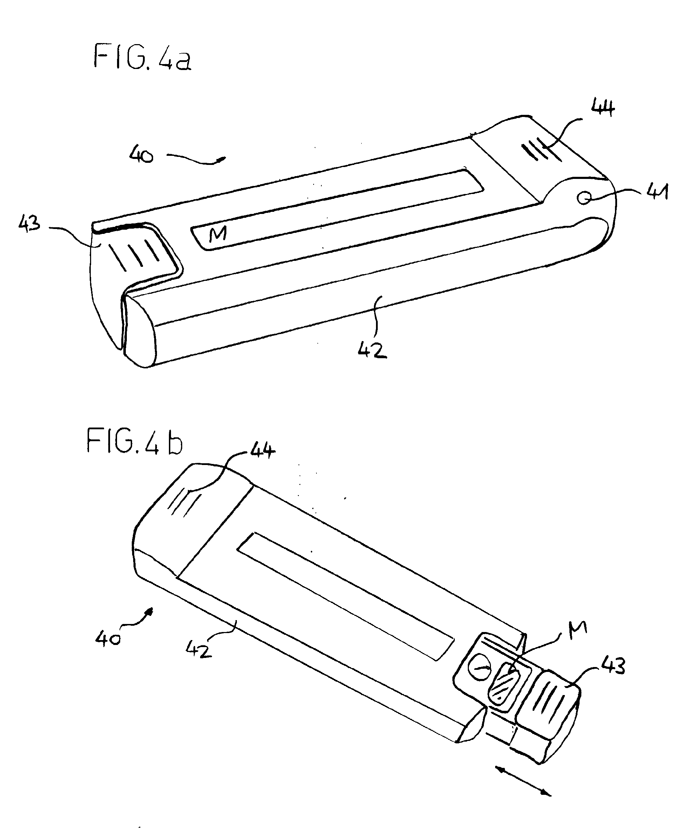

Figures 4a and 4b schematically show a medicine container 40. The medicine container 40 comprises a housing 42. The housing 42 is provided with an infrared communication interface 41. The housing 42 is further provided with a loud speaker 44. The loud speaker 44 may create an alarm reminding the patient to take his or her medicine. Medicine M is stored in a drawer 43 which is inserted into a channel in the housing 42. When the patient should take his or her medicine, an alarm sounds on the loud speaker 44. For taking the medicine M the patient removes the drawer 43 from the housing 42. Removal of the drawer 43 from the housing 42 is detected e.g. by means of an electrical contact and corresponding compliance data are stored in a memory (not shown) in the housing 42. Compliance data can be transmitted (see figure 2b) to the terminal device by means of the infrared communication interface 41. At the end of a day, all medicine M has been taken and the patient completely removes the drawer (which is empty) from the housing 42 and inserts a new drawer for the next day.

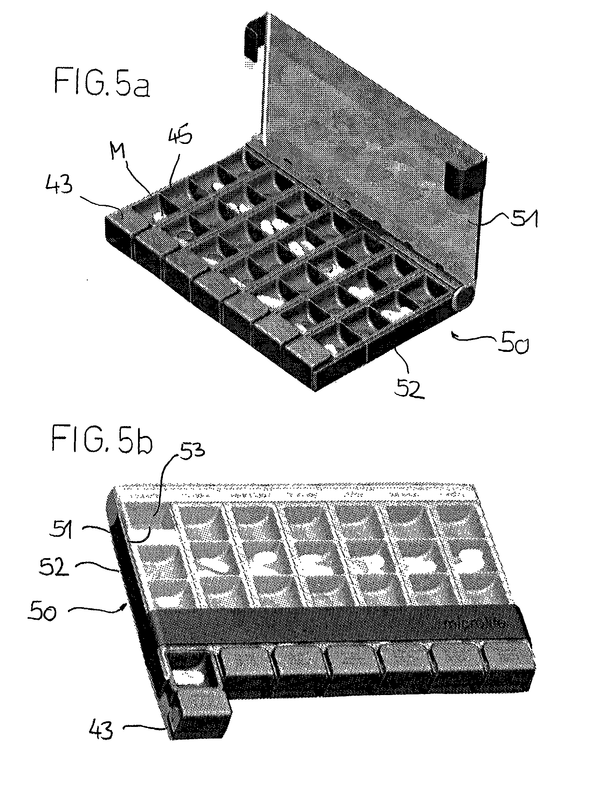

Figure 5a and 5b show a holder 50 for storing drawers 43 containing medicine M for each day. The holder 50 has a housing 52 in which channels 53 are formed, into which the drawers 43 can be inserted. The holder 50 is closed with a cover 51. For filling the drawers 43, medicine M is inserted into compartments formed in the drawers 43. When the compartments 45 are completely filled, the cover 51 is closed. Each day, the patient removes one drawer 43 from the holder 50 as shown in figure 5b. The drawer 43 is then inserted into the housing 42 of the medicine container 40 as shown in figure 4a and 4b.

The blood pressure in the terminal device 20 as shown in figures 3a, 3b is measured and determined in a conventional manner, e.g. in the oscillometric method. The terminal device 20 can also be used as a acquisition, storage or treatment device for medical parameter data measured with another medical parameter sensor, e.g. the temperature measured with a fever thermometer, the body weight determined with a body weight scale or the glucose level in the blood measured with a glucose meter. If such devices are provided with a conventional infrared communication port (not shown), such data can be easily transmitted to and stored in the terminal device 20.

## Claims

1. A system (1) for monitoring medical data (D) of a patient, the system comprising
at least one central computer (10) having a first communication interface (11) and
at least one terminal device (20) for measuring and storing medical data (D) of the patient
wherein the terminal device (20) has a calculating arrangement (21) with means (22) for storing computer readable program means (S) for causing the calculating arrangement to acquire and/or treat and/or store medical data (D),
wherein the terminal device (20) has a second communication interface (23) for exchanging data (C, D, S) and
wherein said computer readable program means (S) can be loaded from the central computer (10) into said means for storing (22) by means of said first and second communication interfaces (11, 23).

2. A system according to claim 1, wherein the system (1) further includes a central server (5) for centrally storing said computer readable program means (S) and wherein said at least one central computer (10) is adapted to access said central server (5) for downloading said computer readable program means (S) to said central computer (10).

3. A system according to one of the claims 1 or 2, wherein said central computer (10) is further adapted to receive said medical data (D) from said terminal device (20) by means of said first and second communication interfaces (11, 23).

4. A system according to one of the claims 1 to 3, wherein said computer readable program means (S) are designed to run on said terminal device (20) for a predetermined period of time.

5. A system according to claim 3 or 4, wherein said central computer (10) is adapted to treat said medical data (D, C) and to generate a report (R) relating to said medical data (D, C).

6. A system according to one of the claims 1 to 5, wherein said terminal device (20) has at least one connector (24b) for removably connecting a connector (24a) of an external sensor (25) for measuring said medical data (D).

7. A system according to claim 6, wherein said terminal device (20) is a blood pressure monitor terminal device comprising a pressure sensor (26) and wherein said connector (24b) is adapted to receive a connector (24a) attached to a tube (27) associated with a blood pressure measuring cuff (25).

8. A system according to one of the claims 1 to 7, wherein said terminal device (20) is adapted to receive data (C) from an additional medical data monitoring device (40).

9. A system according to one of the claims 1 to 8, wherein said terminal device (20) is provided with a touch screen (28) allowing the entry of user instructions and/or the display of medical data (D).

10. A system according to one of the claims 1 to 8, wherein the terminal device (20) is provided with one single operation button (29).

11. A system according to one of the claims 1 to 10, wherein the system further comprises at least one portable medicine container (40), said medicine container (40) comprising a third communication interface (41) adapted to exchange medicine compliance data (C) with said terminal device (20).

12. A system according to claim 11, wherein said medicine container (40) comprises a housing (42) and a drawer (43) for the reception of medicine (M) insertable in said housing (42),
wherein said medicine container (40) has means for detecting withrawal of said drawer (43) from said housing (42).

13. A system according to one of the claims 11 or 12, wherein said medicine container (40) includes alarm means for reminding the patient to take the medicine (M).

14. A system according to one of the claims 12 to 13, wherein the system (1) further includes a holder (50) for receiving a plurality of drawers (43) of a medicine container (40).

15. A terminal device (20) for measuring and storing medical data (D, C) of a patient, comprising
a calculating arrangement (21) with means for storing (22) computer readable program means (S) for causing the calculating arrangement (21) to acquire, treat or store medical data (D),
wherein the terminal device (20) has a communication interface (23) for exchanging data (D, C) with a central computer (10) and
wherein said computer readable program means (S) can be loaded and stored from said central computer (10) into said means for storing (22) by means of said communication interface (23).

16. A device according to claim 15, wherein said terminal device (20) has at least one connector (24b) for removably connecting a connector (24a) of an external sensor (25) for measuring said medical data (D).

17. A device according to claim 16, wherein said terminal device is a blood pressure monitoring terminal device comprising a pressure sensor (26) and
wherein said connector (24b) is adapted to receive a connector (24a) attached to a tube (27) associated with a blood pressure monitor cuff (25).

18. A device according to one of the claims 15 to 17, wherein said terminal device (20) is adapted to receive data (C) from an additional medical data device (40).

19. A device according to one of the claims 15 to 18, wherein said terminal device (20) is provided with a touch screen (21) allowing the entry of user instructions and/or the display of said medical data (D).

20. A device according to one of the claims 15 to 19, wherein said terminal device (20) is provided with one single operation button (29).

21. A medicine container (40) comprising a communication interface (41) adapted to exchange medicine compliance data (C) with a terminal device (20), wherein the medicine container (4) has a housing (42) and a drawer (43) for the reception of medicine (M) insertable in said housing (42) and wherein said medicine container (40) has means for detecting withdrawal of the drawer (43) from said housing (42).

22. A medicine container according to claim 21, wherein said medicine container (40) includes alarm means for reminding the patient to take the medicine (M).

23. A holder (50) for holding medicine (M), comprising a plurality of slot like channels (53) for receiving a drawer (43) for a medicine container, said channels having an open end allowing insertion of said drawer (43) into said channels (53).

24. A holder according to claim 23, wherein said holder (50) has a removable cover (51) and wherein said drawer (43) have a receiving opening for receiving medicine (M) directed towards said cover (51) for supplying medicine (M) into said drawers (43).

25. A method for providing a medical parameter measuring device, comprising the steps of
- providing a terminal device with a calculating arrangement (21) with means (22) for storing computer readable program means (S) and with a communcation interface (23)
- loading and storing computer readable program means (5) in said means (22) for storing by means of said communication interface (23),
said program means (S) causing said terminal device (20) to acquire and/or treat and/or store medical data (O) when run in said calculating arrangement.
